# EUROPÄISCHE PATENTANMELDUNG

(11) **EP 3 851 033 A1**
(43) Veröffentlichungstag der Anmeldung: **21.07.2021**
(21) Anmeldenummer: 21000012.1
(22) Anmeldetag: 15.01.2021
(51) Int. Cl.: A61B 5/022

(54) **ARMAUFLAGE**

(30) Priorität: 15.01.2020 DE 202020000280 U
(71) Anmelder: Fellenberg, Elke, 10409 Berlin (DE)
(72) Erfinder: Fellenberg, Elke, 10409 Berlin (DE)
(74) Vertreter: ETL IP Patent- und Rechtsanwaltsgesellschaft mbH

(57) **Zusammenfassung**

Die Erfindung betrifft eine Armauflage mindestens aufweisend einen Standfuß (1) und eine Unterarmauflage (2), wobei die Unterarmauflage (2) gegenüber dem Standfuß (1) höhenverstellbar und mittels eines Gelenkes (6) unterhalb der Unterarmauflage (2) befindlich mindestens kippbar angeordnet ist und die Unterarmauflage (2) über ein Gelenk (4) mit einer Oberarmauflage (3) verbunden ist, wobei die Längen der Unter- und/oder der Oberarmauflagen (2, 3) so bemessen sind, dass am Oberarm eine Manschette (11) zur Arterienkomprimierung und/oder am Unterarm eine Handgelenkmessgerät anordenbar ist.

## Beschreibung

Die Erfindung betrifft eine Armauflage, die insbesondere beim Blutdruckmessen an einem Oberarm zum Einsatz kommt.

In vielen Arztpraxen erfolgt das Messen des Blutdruckes am Oberarm in einer für den Patienten ergometrisch ungünstigen Haltung, nämlich indem er seinen Arm auf dem Schreibtisch oder einer Liege abstützt, während eine Manschette, welche mit einem Organismus-Kompressions-Fluidbalg ausgestattet ist, um den Oberarm gelegt wird und durch Ausdehnen eine Arterie des Patienten komprimiert.

Bekannt sind weiter Armauflagen, die mit Blutdruckmessgeräten kombiniert sind.

So beschreibt die DE 11 2009 000 630 T5 eine Blutdruckmessgerätstulpe, die ein Oberarmunterstützungsfundament und ein Armband besitzt, wobei das Oberarmunterstützungsfundament aufweist: Eine Oberarmunterstützungsoberfläche, die den Oberarm unterstützt während der Oberarm darauf angebracht wird; einen Wicklungsmechanismus, der innerhalb des Oberarmunterstützungsfundaments angeordnet ist und in der Lage ist, ein Ende in einer longitudinalen Richtung des Armbands in eine Wicklungsrichtung zu ziehen, so dass ein Teil des Armbands in der Nähe des einen Endes gewickelt wird.

Weiter ist aus der DE 11 2009 003 709 T5 ein Blutdruckmessgerät bekannt, das einen Ellenbogenauflage aufweist und das eine nach unten gerichteter Neigung einer Armauflage detektiert und anzeigt.

Nachteilig bei diesen Blutdruckmessgeräten ist, dass sie gewöhnlich wegen des Preises nur in Arztpraxen einsetzbar sind. Auch dürfte der Zeitfaktor der Einstellung das Zeitvolumen der eigentlichen Messung deutlich übersteigen.

Aufgabe der Erfindung ist es, eine Armauflage insbesondere für das Blutdruckmessen am Oberarm und am Handgelenk vorzuschlagen, die sowohl in der Arztpraxis als auch Zuhause einsetzbar ist und keines großen Einstellaufwandes bedarf.

Gelöst wird diese Aufgabe mit den Merkmalen des Anspruches 1. Vorteilhafte Ausgestaltungen sind Gegenstand der Unteransprüche.

Erfindungsgemäß wird eine Armauflage vorgeschlagen, die mindestens einen Standfuß und eine Unterarmauflage aufweist, wobei die Unterarmauflage gegenüber dem Standfuß höhenverstellbar und mittels eines Gelenkes unterhalb der Unterarmauflage befindlich mindestens kippbar angeordnet ist und die Unterarmauflage über ein Gelenk mit einer Oberarmauflage verbunden ist, wobei die Längen der Unter- und/oder der Oberarmauflagen so bemessen sind, dass am Oberarm eine Manschette zur Arterienkomprimierung und/oder am Unterarm eine Handgelenkmessgerät anordenbar ist.

Durch den Standfuß erhält die Armauflage die notwendige Standfestigkeit, wobei der Standfuß auf dem Boden oder auf einer anderen ebenen Fläche aufgestellt werden kann.

Zum Verfahren oder Verschieben verfügt der Standfuß bei einer bevorzugten Ausführung über Räder oder Gleiter. Dabei kann der Standfuß auf dem Boden oder einer ebenen Fläche verschoben werden.

Durch einen Höhenversteller, vorzugsweise einen Teleskopstab, erfolgt die Höhenverstellung zwischen dem Standfuß und der Unterarmauflage angepasst an die Patientengröße in der Sitzposition oder an die Liegehöhe.

Mittel des Gelenkes unterhalb der Unterarmauflage befindlich lässt sich der Unterarm in die richtige Messposition kippen.

Da die Unterarmauflage über ein Gelenk mit einer Oberarmauflage verbunden ist, bekommt auch der Oberarm eine Abstützung.

Der Arm des Patienten ruht so optimal und entspannt in der Armauflage, wenn die Gelenke, der Höhenversteller und soweit vorhanden die Räder festgestellt sind. Die Position des Feststellens sollte dabei stufenlos vornehmbar sein.

Bei einer weiteren vorteilhaften Ausgestaltung sind die die Gelenke und der Höhenversteller motorisch verstellbar. Verfügen die Gelenke und der Höhenversteller dann noch über Positionssensoren verfügen, über die eine optimale Einstellung für einen Patienten erfassbar ist und die Daten abrufbar patientenspezifisch speicherbar in einem Speicher sind, bestehen die Voraussetzungen, dass über eine Eingabeeinrichtung eine Patientenerkennung vorgenommen und die Armauflage automatisch in der optimalen Stellung positioniert wird.

Diese Ausgestaltung ist von großem Vorteil, weil der betreffende Patientenkreis in der Regel bei jedem Arztbesuch einer Blutdruckmessung unterzogen werden. Der Einstellaufwand für die Armauflage entfällt damit völlig.

Eine weitere vorteilhafte Ausgestaltung sieht vor, dass die mindestens die Unterarmauflage eine Polsterung aufweist.

Weiterhin ist es von Vorteil, wenn mindestens die Unterarmauflage schalenförmig ausgebildet ist, so dass der Unterarm auch eine seitliche Abstützung bekommt.

Die schalenförmige Ausbildung der Unterarmauflage wird durch einen festen Körper als Auflage oder durch die im Körper angeordnete Polsterung vorgenommen.

Insbesondere bei der Verwendung von Handgelenkmessgeräten hat es sich als vorteilhaft erwiesen, wenn die Unterarmauflage stirnseitig über Aufnahmen für einen Einsteckbügel verfügt. Die Unterarmauflage lässt sich so verlängern, so dass die Hand beim Messen nicht abknicken kann.

Die Erfindung soll anhand der Zeichnungen erläutert werden. Es zeigen:
- Fig. 1: Die Armabstützung bei einer Messung am Oberarm und
- Fig. 2: bei einer Messung am Handgelenk.

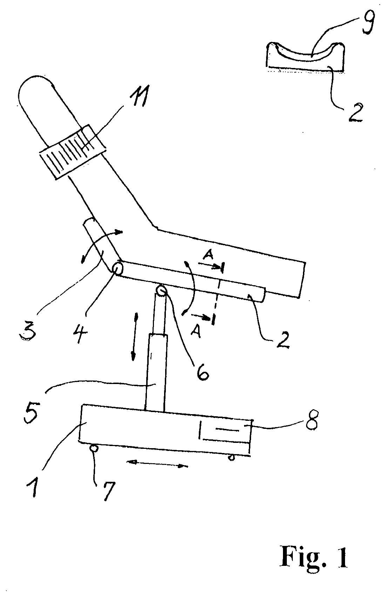

**Fig. 1** zeigt die Armabstützung bei einer Messung am Oberarm.

Die Armauflage weist einen Standfuß 1 und eine Unterarmauflage 2 auf, die durch einen Höhenversteller 5 in Form eines Teleskopstabes miteinander verbunden sind. Mittels eines Gelenkes 6 unterhalb der Unterarmauflage 2 ist die Unterarmauflage 2 mindestens kippbar gegenüber dem Höhenversteller 5 angeordnet.

Die Unterarmauflage 2 weist weiter eine Oberarmauflage 3 auf, die mittels des Gelenkes 4 gegenüber der Unterarmauflage 2 verstellbar ist. Die Länge der Oberarmauflage 3 ist dabei so bemessen, dass am Oberarm bei der Blutdruckmessung eine Manschette 11 zur Arterienkomprimierung angebracht werden kann.

Der Arm des Patienten ist so optimal für die Blutdruckmessung positionierbar. Die so vorgenommene Einstellung wird durch das Fixieren der Räder 7, der Gelenke 4 und 6 sowie des Höhenverstellers 5 stabilisiert.

Die Einstellung kann manuell vorgenommen werden. Für Arztpraxen ist es jedoch vorteilhafter, wenn die Gelenke 4, 6 und der Höhenversteller 5 motorisch verstellbar sind. Durch ein patientenspezifisches Speichern der optimalen Einstellung -erfasst durch Positionssensorenin einem Speicher 8 bestehen dann die Voraussetzungen, dass eine selbsttätige Einstellung vornehmbar ist, wenn der jeweilige Patient über eine entsprechende Eingabe im Speicher 8 aufgerufen wird.

Weiter wird gezeigt, dass die Unterarmauflage 2 über eine Polsterung 9 verfügt, die der schalenförmigen Ausbildung der Unterarmauflage 2 folgt. Möglich ist es auch, eine ebene Unterarmauflage zu verwenden und die Schalenform durch die Polsterung 9 vorzunehmen oder eine Kombination zu wählen. Es versteht sich dabei, dass mindestens die Polsterung abwaschbar und desinfizierbar ist.

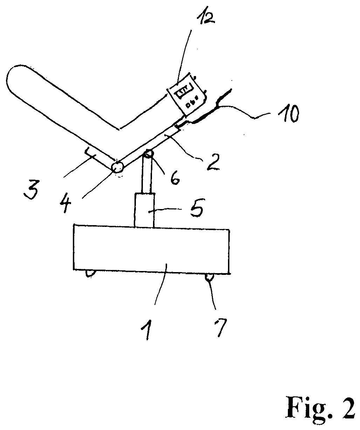

Die Darstellung in **Fig. 2** unterscheidet sich von der vorangegangenen dadurch, dass hier eine Messung am Unterarm vorgenommen wird. Dabei kommt der Höhenlage des Handgelenkmessgerätes 12 eine besondere Bedeutung zu - es muss sich in Herzhöhe bei der Messung befinden. Das ist mittels der Höheneinstellung und des Gelenkes 6 zwischen dem Höhenversteller 5 und der Unterarmauflage 2 kein Problem.

Wie in der Darstellung gezeigt, stützt die Oberarmablage 3 das Ellenbogengelenk des Patienten ab, so dass der komplette Arm in entspannter Lage auf der Armauflage ruht.

Damit das Handgelenk bei der Messung nicht abknickt, ist weiter vorgesehen, dass die Unterarmauflage 2 stirnseitig Aufnahmen für einen Einsteckbügel 10 aufweist, auf dem dann die Hand aufliegt.

Gewöhnlich werden derartige Handgelenkmessgeräte 12 im Privathaushalt eingesetzt, d. h. eine Anpassung an unterschiedliche Patienten ist nur in Ausnahmefällen notwendig. Deshalb dürfte bei dieser Ausführung die manuelle Einstellung und Fixierung der Gelenke 4, 6 und des Höhenverstellers 5 ausreichend sein, was natürlich die Kosten für die Armauflage deutlich senkt. Das schließt aber die motorische Verstellbarkeit nicht aus.

Mit der vorgeschlagenen Armabstützung sind sowohl Oberarmmessungen als auch Handgelenkmessungen des Blutdruckes in einer entspannten Lage für den Nutzer gegeben, mit der Folge, dass sich die Genauigkeit der Messwerte erhöht.

Neben der Blutdruckmessung ist eine derartige Armaüflage auch bei der Blutentnahme vorteilhaft einsetzbar.

### Bezugszeichenliste

- 1: Standfuß
- 2: Unterarmauflage
- 3: Oberarmauflage
- 4: Gelenk zwischen Unter- und Oberarmauflage
- 5: Höhenversteller
- 6: Gelenk
- 7: Räder/Gleiter
- 8: Speicher
- 9: Polsterung
- 10: Einsteckbügel
- 11: Manschette
- 12: Handgelenkmessgerät

## Patentansprüche

1. Armauflage mindestens aufweisend einen Standfuß (1) und eine Unterarmauflage (2), wobei die Unterarmauflage (2) gegenüber dem Standfuß (1) höhenverstellbar und mittels eines Gelenkes (6) unterhalb der Unterarmauflage (2) befindlich mindestens kippbar angeordnet ist und die Unterarmauflage (2) über ein Gelenk (4) mit einer Oberarmauflage (3) verbunden ist, wobei die Längen der Unter- und/oder der Oberarmauflagen (2, 3) so bemessen sind, dass am Oberarm eine Manschette (11) zur Arterienkomprimierung und/oder am Unterarm eine Handgelenkmessgerät anordenbar ist.

2. Armauflage nach Anspruch 1, **dadurch gekennzeichnet, dass**
zur Höhenverstellung zwischen dem Standfuß (1) und der Unterarmauflage (2) ein Höhenversteller (5), vorzugsweise ein Teleskopstab, eingesetzt ist.

3. Armauflage nach Anspruch 1 oder 2, **dadurch gekennzeichnet, dass**
der Standfuß (1) zum Verfahren und Verschieben über Räder (7) oder Gleiter verfügt.

4. Armauflage nach einem der Ansprüche 1 bis 3,
**dadurch gekennzeichnet, dass**
die Gelenke (4, 6), der Höhenversteller (5) und/oder die Räder (7) feststellbar sind, vorzugsweise stufenlos.

5. Armauflage nach einem der Ansprüche 1 bis 4,
**dadurch gekennzeichnet, dass**
die Gelenke (4, 6) und/oder der Höhenversteller (5) motorisch verstellbar sind.

6. Armauflage nach einem der Ansprüche 1 bis 5,
**dadurch gekennzeichnet, dass**
die Gelenke (4, 6) und/oder der Höhenversteller (5) über Positionssensoren verfügen, über die eine optimale Einstellung für einen Patienten erfassbar ist und die Daten abrufbar patientenspezifisch speicherbar in einem Speicher (8) sind.

7. Armauflage nach Anspruch 6, **dadurch gekennzeichnet, dass**
der Speicher (8) über eine Eingabeeinrichtung zur Patientenerkennung verfügt.

8. Armauflage nach einem der Ansprüche 1 bis 7,
**dadurch gekennzeichnet, dass**
die Unterarmauflage (2) eine Polsterung (9) aufweist.

9. Armauflage nach einem der Ansprüche 1 bis 8,
**dadurch gekennzeichnet, dass**
mindestens die Unterarmauflage (2) schalenförmig ausgebildet ist.

10. Armauflage nach Anspruch 9, **dadurch gekennzeichnet, dass**
die schalenförmige Ausbildung der Unterarmauflage (2) durch einen festen Körper als Auflage oder durch die im Körper angeordnete Polsterung (9) vorgenommen ist.

11. Armauflage nach einem der Ansprüche 1 bis 10,
**dadurch gekennzeichnet, dass**
die Unterarmauflage (2) stirnseitig Aufnahmen für einen Einsteckbügel (10) verfügt.
